# EUROPEAN PATENT APPLICATION

(11) **EP 2 457 523 A2**
(43) Date of publication of application: **30.05.2012**
(21) Application number: 11250781.9
(22) Date of filing: 09.09.2011
(51) Int. Cl.: A61B 17/34

(54) **Access device including expanding distal flange**

(30) Priority: 24.11.2010 US 416781 P; 02.05.2011 US 481316 P; 29.07.2011 US 193647
(71) Applicant: Tyco Healthcare Group LP, New Haven, CT 06511 (US)
(72) Inventor: Pribanic, Russell, Roxbury, CT 06783 (US)
(74) Representative: Soames, Candida Jane

(57) **Abstract**

A surgical apparatus for positioning within a tissue tract accessing an underlying body cavity including a compressible seal anchor member. The seal anchor member includes a leading portion that is expandable to facilitate transitioning between a first condition and a second condition. Insertion of the seal anchor member into a tissue tract is facilitated by the reduced dimensions of the leading portion while in the first condition. Anchoring of the seal anchor member within the tissue tract is facilitated by the expanded dimensions of the leading portion while in the second condition.

## Description

### CROSS REFERENCE TO RELATED APPLICATION

The present application claims the benefit of and priority to U.S. Provisional Application Serial No. 61/416,781 filed on November 24, 2010, and U.S. Provisional Application 61/481,316 filed on May 2, 1011, the entire contents of which are incorporated herein by reference.

### BACKGROUND

### Technical Field

The present disclosure relates generally to an access device for use in a surgical procedure. More particularly, the present disclosure relates to a seal anchor member adapted for insertion into an incision, or a naturally occurring bodily orifice, in tissue, and for the sealed reception of one or more surgical objects such that a substantially fluid-tight seal is formed with both the tissue and the surgical object or objects.

### Background of Related Art

A minimally invasive surgical procedure is one in which a surgeon enters a patient's body through a small opening in the skin or through a naturally occurring opening (*e.g.*, mouth, anus, or vagina). Such procedures have several advantages over traditional open surgeries. In particular, as compared to traditional open surgeries, minimally invasive surgical procedures result in reduced trauma and recovery time for patients. Generally, such procedures are referred to as "endoscopic", unless performed on the patient's abdomen, in which case the procedure is referred to as "laparoscopic". Throughout the present disclosure, the term "minimally invasive" should be understood to encompass both endoscopic and laparoscopic procedures.

During a typical minimally invasive procedure, surgical objects, such as surgical access devices (*e.g.*, trocar and cannula assemblies) or endoscopes, are inserted into the patient's body through the incision in tissue. In general, prior to the introduction of the surgical object into the patient's body, insufflation gases are used to enlarge the area surrounding the target surgical site to create a larger, more accessible work area. Accordingly, the maintenance of a substantially fluid-tight seal is desirable so as to prevent the escape of the insufflation gases and the deflation or collapse of the enlarged surgical site.

To this end, various valves and seals are used during the course of minimally invasive procedures and are widely known in the art. However, a continuing need exists for a seal anchor member that can be inserted directly into the incision in tissue and that can accommodate a variety of surgical objects while maintaining the integrity of an insufflated workspace.

### SUMMARY

Disclosed herein is a surgical apparatus for positioning within a tissue tract accessing an underlying body cavity. The surgical apparatus includes a compressible seal anchor member including a leading portion, a trailing portion, and an intermediate portion disposed between the leading and trailing portions. Two or more lumens longitudinally extend between the leading and trailing portions. The one or more lumens are configured and adapted to receive instrumentation therein in a substantially sealed relation. Associated with the leading portion is a flange that is transitionable between a first condition and a second condition. In particular, the flange is expandable to transition from a first dimension to a second dimension. A conduit may be in fluid communication with the flange associated with the leading portion. A source of fluid (*e.g.*, gas or liquid) may be operably coupled to the conduit. In an embodiment, a plunger may be translated through the conduit to force a volume of fluid within the conduit into the flange associated with the leading portion.

While the flange associated with the leading portion is in the first condition, the seal anchor member is placed within a tissue tract. The seal anchor member is anchored within the tissue tract by expanding the flange associated with the leading portion, and transitioning the flange to a second dimension. Surgical objects are inserted within the lumens, and a surgical procedure is performed. Once the surgical procedure is completed, the surgical objects may be removed. Removal of the seal anchor member is facilitated by contracting the flange associated with the leading portion to transition the flange back to its first dimension, *e.g.*, its initial width and diameter.

These and other features of the apparatus disclosed herein will become more readily apparent to those skilled in the art from the following detailed description of various embodiments of the present disclosure.

### BRIEF DESCRIPTION OF THE DRAWINGS

Various embodiments of the present disclosure are described hereinbelow with reference to the drawings, wherein:

Fig. 1 is a perspective view of a seal anchor member in accordance with the principles of the present disclosure shown in a first condition positioned relative to tissue;

Fig. 2 is a cross-sectional view of the seal anchor member of Fig. 1 taken along section-line 2-2 of Fig. 1 illustrating a port that extends longitudinally therethrough;

Fig. 3 is a view of the port of Fig. 2 with a surgical object inserted therethrough;

Fig. 4A is a perspective view of the seal anchor member of Fig. 1 shown in a first condition;

Fig. 4B is a perspective view of the seal anchor member of Fig. 1 shown in a second condition;

Fig. 5 is a bottom perspective view of the seal anchor member of Fig. 1 shown in the second condition;

Fig. 5A is a bottom perspective view of the seal anchor member of Fig. 1 shown operably coupled to a source of fluid;

Fig. 5B is a side view of a of the seal anchor member of Fig. 1 shown with a plunger member in a first position relative to the seal anchor member;

Fig. 5C is a side view of the seal anchor member of Fig. 1 shown with a plunger member in a second position relative to the seal anchor member;

Fig. 6A is a perspective view of the seal anchor member of Fig. 1 shown in the first condition and positioned within a tissue tract; and

Fig. 6B is a perspective view of the seal anchor member of Fig. 1 shown in the second condition and positioned within the tissue tract.

### DETAILED DESCRIPTION OF EMBODIMENTS

In the figures and in the description that follows, in which like reference numerals identify similar or identical elements, the term "proximal" will refer to the end of the apparatus that is closest to the clinician during use, while the term "distal" will refer to the end that is farthest from the clinician, as is traditional and known in the art.

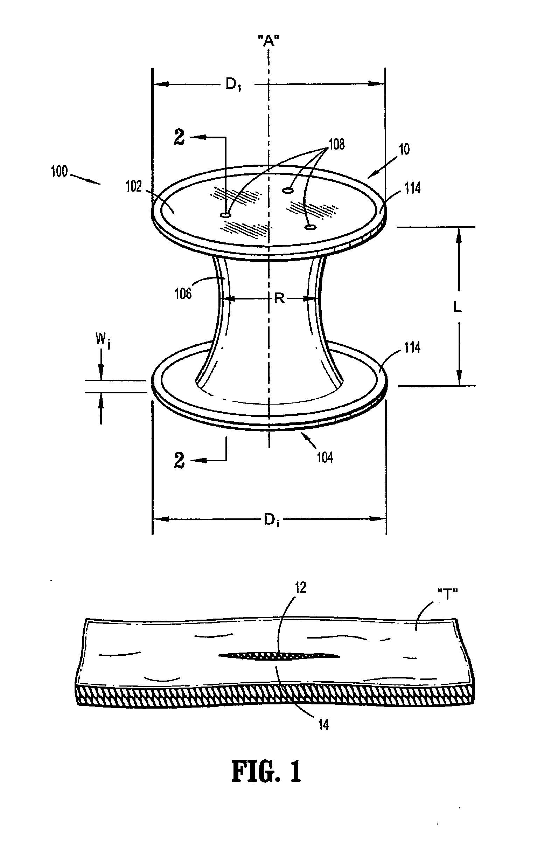

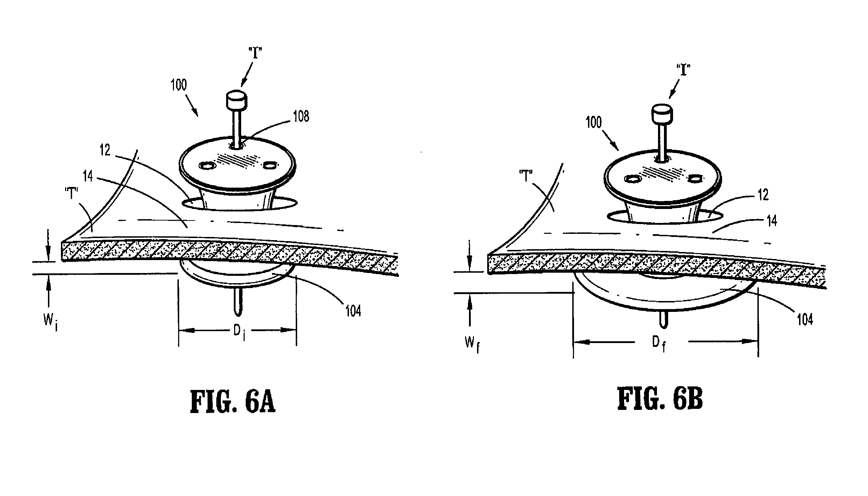

With reference to Figs. 1-6B, a seal anchor member 100 will now be described. Seal anchor member 100 defines a longitudinal axis "A" and includes respective trailing (proximal) and leading (distal) portions 102, 104, and an intermediate section 106 disposed between the trailing and leading portions 102, 104. Seal anchor member 100 includes one or more ports 108 that extend generally longitudinally between trailing and leading portions 102, 104, respectively, and through seal anchor member 100.

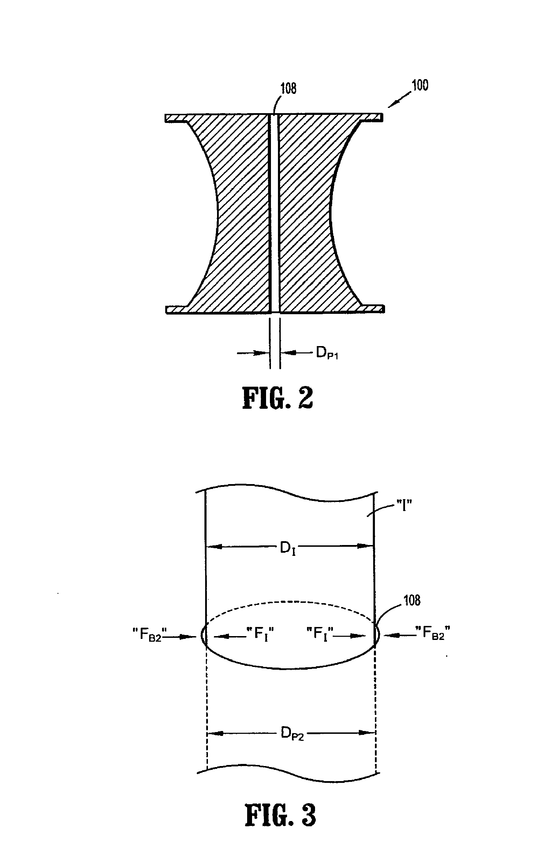

The seal anchor member 100 may be substantially formed from a suitable foam material having sufficient compliance to form a seal about one or more surgical objects, shown generally as surgical object "I" (Fig. 3), and also establish a sealing relation with the tissue. The foam is preferably sufficiently compliant to accommodate off axis motion of the surgical object "I". In one embodiment, the foam includes a polyisoprene material.

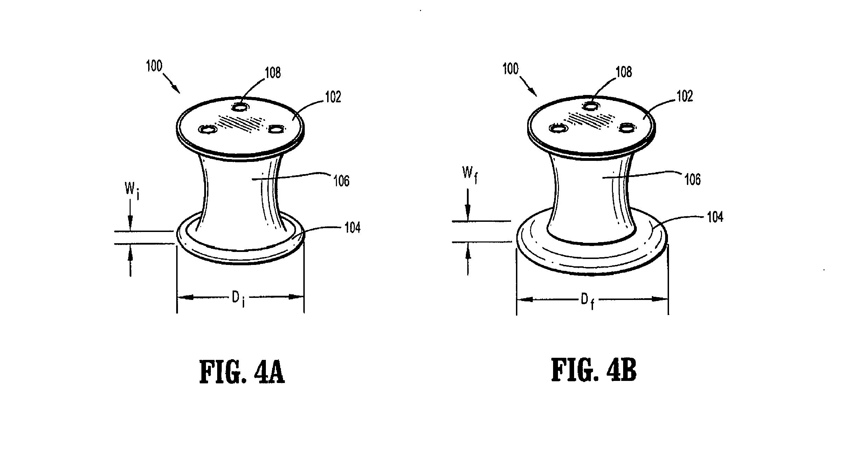

The trailing portion 102 includes a flange 114a, and defines a first diameter D₁ The leading portion 104 includes a flange 114b, and defines a diameter that is transitionable between an initial width Wᵢ and diameter Dᵢ in a first condition, *e.g.*, unexpanded or deflated state, (Figs. 1, 4A, and 6A) and a final width, W_{f} and diameter D_{f}, in a second condition, *e.g.*, expanded or inflated state, (Figs. 4B, 5, and 6B). In the first condition, the first diameter D₁ of the proximal portion 102 may be substantially equivalent to the initial diameter Dᵢ of the leading portion 104.

In the first condition, as shown in Fig. 1, the trailing and leading portions 102, 104 define substantially planar surfaces. However, in other embodiments, either or both the trailing and leading portions 102, 104 may define surfaces that are substantially convex to facilitate the insertion of the seal anchor member 100 within tissue tract 12 defined by tissue surfaces 14 and formed in tissue "T", *e.g.*, an incision or naturally occurring bodily orifice.

The intermediate portion 106 defines a radial dimension R and extends longitudinally between the leading portion 104 and the trailing portion 102 to define an axial dimension or length L. The radial dimension R of intermediate portion 106 varies along the length L. Accordingly, seal anchor member 100 defines a cross-sectional dimension that varies along length L, thereby facilitating anchoring of the seal anchor member 100 within tissue "T". In other embodiments, however, the radial dimension R of the intermediate portion 106 may remain substantially uniform along its length L.

The radial dimension R of the intermediate portion 106 may be appreciably less than the diameters of the proximal and distal portions 102, 104 in both the first and second conditions. Therefore, the seal anchor member 100 may define an "hour-glass" shape or configuration to facilitate anchoring of the seal anchor member 100 within the tissue "T". However, in other embodiments, the radial dimension R of the intermediate portion 106 may be substantially equivalent to the diameters of the proximal and distal portions 102, 104. In cross-section, the intermediate portion 106 may exhibit any suitable configuration, *e.g.*, a substantially circular, oval, or oblong shape.

Each port 108 is configured to removably receive the surgical object "I" in a substantially sealed relation. As shown in Fig. 2, prior to the insertion of the surgical object "I", port 108 is in a first state in which the port 108 defines a first or initial dimension D_{P1}. D_{P1} will generally be about 0mm such that the escape of insufflation gas (not shown) through port 108 of seal anchor member 100 in the absence of surgical object "I" is inhibited. For example, the port 108 may be a slit extending longitudinally through intermediate portion 106 and through the trailing and leading portions 102, 104. As shown in Fig. 3, the introduction of the surgical object "I" through the port 108 transitions the port 108 to a second state in which the port 108 defines a second, larger dimension D_{P2} that substantially approximates the diameter D_{I} of the surgical object "I" to facilitate forming a substantially fluid tight seal, between the port 108 and the surgical object "I", to inhibit the escape of insufflation gas through the port 108 in the presence of surgical object "I" inserted therein.

In particular, upon the introduction of surgical object "I" through port 108 as depicted in Fig. 3, the surgical object "I" exerts a force "F_{I}" upon port 108 that is directed radially outward. Force "F_{I}" acts to enlarge the dimensions of port 108 and thereby transition port 108 into the second state thereof in which port 108 defines a second, larger dimension D_{P2} that substantially approximates the diameter D_{I} of surgical object "I". Consequently, an internal biasing force "F_{B}" is created that is directed radially inward, in opposition to force "F_{I}". Internal biasing force "F_{B}" endeavors to return port 108 to reduce the internal dimension of port 108 and thereby return port 108 to the first state thereof. Internal biasing force "F_{B}" is exerted upon surgical object "I" and acts to create a substantially fluid-tight seal therewith. Although port 108 is shown and described as being biased toward an initially closed state, in other embodiments the port 108 may have an initially open state.

D_{I}, and thus D_{P2}, will generally lie within the range of about 5mm to about 12mm, as these dimensions are typical of the surgical objects used during the course of minimally invasive procedures. However, a seal anchor member 100 including a port 108 that is capable of exhibiting substantially larger, or smaller, dimensions in the second state thereof is not beyond the scope of the present disclosure. In addition, seal anchor 100 may be devoid of ports 108. With this arrangement, ports 108 are created within seal anchor member 100 during the insertion of the surgical object "I". In accordance with this embodiment, seal anchor member 100 is formed of a flowable or sufficiently compliable material such as a foam material, *e.g.*, an open-cell polyurethane foam or a gel.

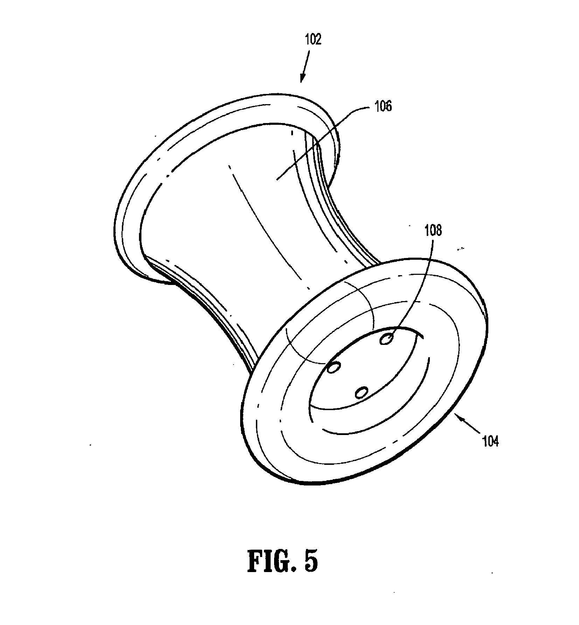

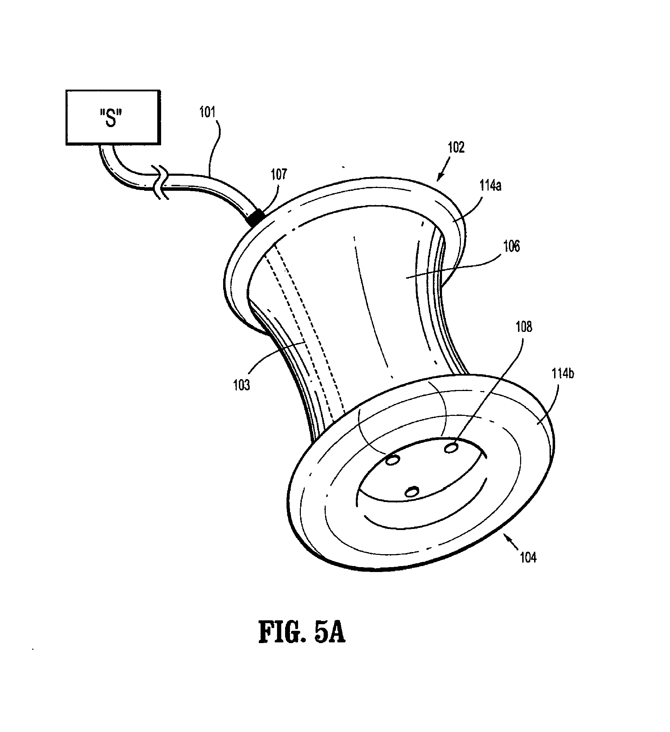

The flange 114b is associated with the leading portion 104 of the seal anchor member 100. As shown best in Fig. 5A, a conduit 103 extends from the trailing portion 102 and terminates within the flange 114b. The conduit 103 may be adapted to receive a tube 101 therein that is operably coupled to a source "S" of fluid. A clamp or a valve 107 may be operably coupled with the tube 101 1 to selectively inhibit the escape of fluid through the conduit 101. The conduit 103 may receive the tube 101 in a substantially sealed relation to inhibit the escape of fluid through the conduit 103.

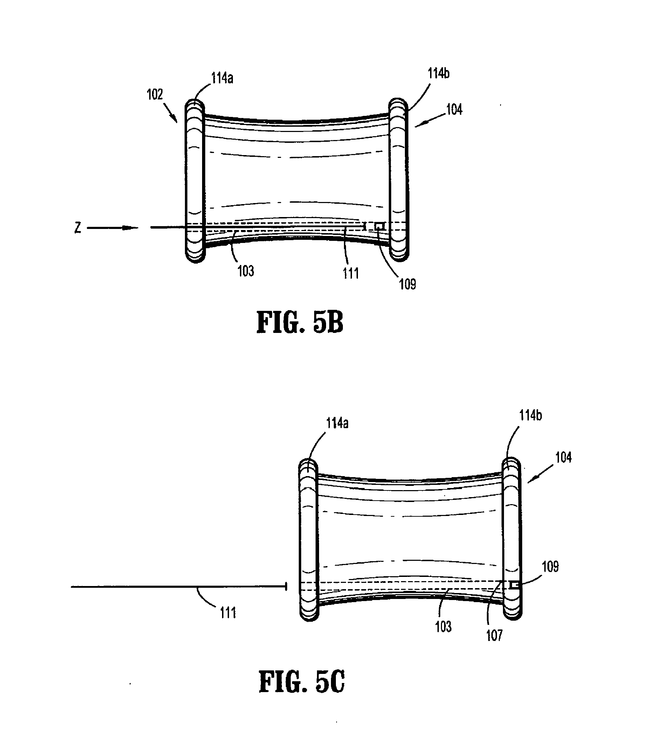

During use, the tube 101 may be inserted into the conduit 103. After placement of the tube 101 into the conduit 103, fluid may be pumped into the flange 114b associated with the distal portion 104. The pumping of fluid into the flange 114b enlarges the dimensions of the distal portion 104 from an initial width Wᵢ and diameter Dᵢ in a first condition (Figs. 1, 4A, and 6A) and a final width Wᵢ and diameter D_{f} in a second condition (Figs. 4B, 5, and 6B). As shown best in Fig. 5, in second condition, *e.g.*, the expanded state, the flange 114b may have a substantially toroidal shape.

Contraction or deflation of the flange 114b may be achieved by reversing the flow of fluid from the source "S", *e.g.*, by applying a suctional force, or by removing the tube 101 and permitting the fluid to flow from the flange 114b proximally through the conduit 103 and out of the proximal region 102.

Alternatively, as shown in Figs. 5B and 5C, a plug 109 may be placed within the conduit 103. The plug 109 is configured and adapted to translate through the length of the conduit 103. As shown in Fig. 5B, a plunger 111 may be distally translated, *e.g.*, in the direction of arrow Z, through the conduit 103, thereby forcing the volume of air contained within the conduit 103 into the flange 114b. Contraction of the flange 10 may be achieved by further distally translating the plug 109 until it exits the conduit 103 and enters the interior of the flange 114b. Once the plug 109 is within the flange 114b, the air or fluid within the flange 114 is free to exit the flange 114b through the conduit 103, thereby facilitating the contraction of the flange 114 back toward its initial width Wᵢ and diameter Dᵢ. Alternatively, the plunger 111 may be operably coupled to the plug 109, and may be proximally translated through the conduit 103 thereby contracting the flange 114b. A protrusion 107 at or near the distal end of the conduit 103 may provide a tactile depth indication by providing resistance to the continued distal translation of the plug 109 by the plunger 111. Continued distal advancement of the plug 109 by the plunger 111 would necessitate overcoming the resistance provided by the protrusion 107. In an embodiment, the conduit 103 may be visible from outside of the seal anchor member 100.

The use and operation of the seal anchor member 100 during a surgical procedure will now be described with reference to Figs. 6A and 6B. As shown in Fig. 6A, the seal anchor member 100 is inserted into tissue tract 12 within tissue "T" while the seal anchor member 100 is in its initial state, *e.g.*, the flange 114b of the distal portion 104 has an initial diameter Dᵢ and an initial width Wᵢ. Once placed within the tissue tract 12, anchoring of the seal anchor member 100 within the tissue tract 12 is achieved by expanding the flange 114b, in a manner as described above, to transition the flange 114b to a final width W_{f} and diameter D_{f to} facilitate anchoring of the seal anchor member 100 within the tissue tract 12. The surgical objects "I" may be inserted prior to or after expansion of the flange 114b. Once the surgical procedure is complete, the flange 114 is contracted and transitioned back to its initial diameter Dᵢ and initial width Wᵢ to facilitate removal of the seal anchor member 100 from the tissue tract 12.

Although the illustrative embodiments of the present disclosure have been described herein with reference to the accompanying drawings, the above description, disclosure, and figures should not be construed as limiting, but merely as exemplifications of particular embodiments. It is to be understood, therefore, that the disclosure is not limited to those precise embodiments, and that various other changes and modifications may be effected therein by one skilled in the art without departing from the scope or spirit of the disclosure.
The invention may be described by reference to the following numbered paragraphs:
1. A surgical apparatus for positioning within a tissue tract accessing an underlying body cavity, comprising:
   a compressible seal anchor member comprising:
      a leading portion;
      a trailing portion;
      an intermediate portion disposed between the leading and trailing portions; and
      two or more lumens longitudinally extending between the leading and trailing
      portions,
   wherein the leading portion includes a flange, the flange expandable to transition between a first dimension and a second dimension.
2. The surgical apparatus of paragraph 1, further comprising a conduit in fluid communication with the flange associated with the leading portion.
3. The surgical apparatus of paragraph 2, further comprising a source of inflation fluid operably coupled to the conduit.
4. The surgical apparatus of paragraph 2, further comprising a plunger, the plunger translatable through the conduit to force a volume of fluid within the conduit into the flange associated with the leading portion.
5. A method of performing surgery, comprising the steps of:
   providing a compressible seal anchor member including:
      a leading portion;
      a trailing portion;
      an intermediate portion disposed between the leading and trailing portions; and
      two or more lumens longitudinally extending between the leading and trailing portions, wherein the leading portion includes a flange, the flange expandable to transition between a first condition and a second condition, the flange having a first dimension while in the first condition, and a second dimension while in the second condition;
   placing the seal anchor member within a tissue tract while the flange is in the first condition;
   expanding the flange associated with the leading portion to transition the flange to the second condition, thereby anchoring the seal anchor member within the tissue tract;
   inserting one or more surgical objects into the two or more lumens to perform a surgical procedure;
   removing the one or more surgical objects after completing the surgical procedure;
   contracting the flange associated with the leading portion to transition the flange to back to the first condition; and
   removing the seal anchor member from the tissue tract.

## Claims

1. A surgical apparatus for positioning within a tissue tract accessing an underlying body cavity, comprising:
a compressible seal anchor member comprising:
a leading portion;
a trailing portion;
an intermediate portion disposed between the leading and trailing portions; and
two or more lumens longitudinally extending between the leading and trailing
portions,
wherein the leading portion includes a flange, the flange expandable to transition between a first dimension and a second dimension.

2. The surgical apparatus of claim 1, further comprising a conduit in fluid communication with the flange associated with the leading portion.

3. The surgical apparatus of claim 2, further comprising a source of inflation fluid operably coupled to the conduit.

4. The surgical apparatus of claim 2 or claim 3, further comprising a plunger, the plunger translatable through the conduit to force a volume of fluid within the conduit into the flange associated with the leading portion.

5. A compressible seal anchor member according to any preceding claim and including:
a leading portion;
a trailing portion;
an intermediate portion disposed between the leading and trailing portions;
and
two or more lumens longitudinally extending between the leading and
trailing portions, wherein the leading portion includes a flange, the flange expandable to transition between a first condition and a second condition,
the flange having a first dimension while in the first condition, and a second dimension while in the second condition; for use in a method for performing a surgical procedure, wherein the method comprises the step of,
placing the seal anchor member within a tissue tract while the flange is in the first condition;
expanding the flange associated with the leading portion to transition the flange to the second condition, thereby anchoring the seal anchor member within the tissue tract;
inserting one or more surgical objects into the two or more lumens to perform a surgical procedure;
removing the one or more surgical objects after completing the surgical procedure;
contracting the flange associated with the leading portion to transition the flange to back to the first condition; and
removing the seal anchor member from the tissue tract.
